# NEW EUROPEAN PATENT SPECIFICATION

(11) **EP 1 328 286 B2**
(45) Date of publication and mention of the opposition decision: **31.10.2012**
(45) Mention of the grant of the patent: 10.03.2010
(21) Application number: 01970387.5
(22) Date of filing: 27.09.2001
(51) Int. Cl.: A61K 35/20, A61P 19/10, A23C 9/00, A23L 1/305, A23J 3/08

(54) **BONE HEALTH COMPOSITIONS DERIVED FROM ACIDIC WHEY**
ZUSAMMENSETZUNGEN AUS MILCH ZUR FÖRDERUNG DER KNOCHENGESUNDHEIT
COMPOSITIONS POUR LA SANTE OSSEUSE DERIVEES DU LAIT

(30) Priority: 05.10.2000 NZ 50733500
(43) Date of publication of application: 23.07.2003
(73) Proprietor: NEW ZEALAND DAIRY BOARD, Wellington 1 (NZ)
(72) Inventor: REID, Ian, Reginald, Auckland (NZ); CORNISH, Jill, Auckland (NZ); HAGGARTY, Neill, Ward, Palmerston North (NZ); PALMANO, Kay Patricia, Palmerston North (NZ)
(74) Representative: Barton, Matthew Thomas
(86) International application number: PCT/NZ2001/000200
(87) International publication number: WO 2002/028413

(56) References cited:
- WO-A-99/33415
- GB-A- 2 188 526
- DATABASE BIOSIS BIOSCIENCES INFORMATION SERVICE, PHILADELPHIA, PA, US; 1993, SORENSEN E. ET AL.: "Purification and characterization of 3 proteins isolated from the proteose peptone fraction of bovine milk." XP002288567 Database accession no. PREV199396027413
- SORENSEN E. ET AL.: "The location and multimeric nature of component PP3 in bovine milk : purification and characterization of PP3 from caprine and ovine milks." J. DAIRY SCI., vol. 80, 1997, pages 3176-3181, XP002288565
- BAYLESS K. ET AL.: "Isolation and biologial properties of osteopontin from bovine milk." PROTEIN EXPRESSION AND PURIFICATION., vol. 9, 1997, pages 309-314, XP002288566
- PATENT ABSTRACTS OF JAPAN & JP 09 110 896 A (YOTSUBA NYUGYO KK) 28 April 1997
- PATENT ABSTRACTS OF JAPAN & JP 05 246 882 A (SNOW BRAND MILK PROD. CO. LTD.) 24 September 1993
- PATENT ABSTRACTS OF JAPAN & JP 04 108 529 A (YOTSUBA NYUGYO KK) 09 April 1992
- FITZGERALD R.J.: 'Potential uses of caseinophosphopeptides' INT. DAIRY JOURNAL vol. 8, 1998, pages 451 - 457, XP002968208
- SORENSEN E.S. ET AL: 'Posttranslational modifications of bovine osteopontin: identification of twenty-eight phosphorylation and three O-glycosylation sites' PROTEIN SCIENCE vol. 4, 1995, pages 2040 - 2049, XP002968209
- SORENSEN E.S. ET AL: 'Phosphorylation, glycosylation and amino acid sequence of component PP3 from the proteose peptone fraction of bovine milk' JOURNAL OF DIARY RESEARCH vol. 60, 1993, pages 535 - 542, XP002968210

## Description

### FIELD OF THE INVENTION

The invention relates to bone health compositions derived from an acidic protein fraction of milk and in particular to bone health compositions derived from an acidic protein fraction of whey, to a method of producing said bone health compositions, and to medicinal uses of said bone health compositions.

### BACKGROUND TO THE INVENTION

### Bone Physiology and Disease

One of the most prevalent and costly bone diseases is osteoporosis, characterised by a gradual thinning and weakening of the bones. If this deterioration goes untreated, bones are likely to break or fracture with very little trauma.

Although the process of bone loss begins gradually in the mid to late thirties, it is so slow that it may take many years before a sufferer becomes aware of it. Women, generally, are at greater risk of developing osteoporosis than men. This is because, following the menopause, women experience a rapid loss of bone from the skeleton due to the decrease in estrogen production.

Until a person is around 40, the process of breaking down (resorption) and building up (reforming) bone by osteoclasts and osteoblasts respectively is a nearly perfectly coupled system, with one phase stimulating the other. Bone comprises an extracellular protein matrix (mostly collagen fibrils) interspersed with bone cells (osteocytes) with a mineral component laid on to this consisting of calcium salts and other minerals including sodium, magnesium and fluoride. Osteoclasts resorb bone at a particular site and then undergo programmed cell death. Osteoblasts replace the protein matrix (osteoid) and mediate its remineralisation. During remineralisation, the osteoblasts are encased within the calcified material and become osteocytes, cells that help maintain the structure of the bone. Bone turnover mediated by the osteoclasts and osteoblasts occurs throughout life and is known as "remodelling". Estrogen deficiency is believed to delay the programmed cell death or apoptosis of osteoclasts thus leading to a net bone loss.

As a person ages, the remodelling system breaks down and the two processes (resorption and reformation) become out of synchronisation. The reasons for this are not clear. Some individuals have a very high turnover rate of bone; some have a very gradual turnover, but the breakdown of bone eventually overtakes the build-up. Because the patterns of reforming and resorbing bone often vary from patient to patient, experts believe a number of different factors account for this problem. Important hormones, such as estrogen, parathyroid hormone, vitamin D, and blood factors that affect cell growth are involved in this process. Changes in the levels of any of these factors could play a role in the development of osteoporosis.

Post-menopausal women often undergo Hormone Replacement Therapy (HRT) to compensate for reducing natural estrogen levels. HRT is usually not prescribed before a patient is very close to the critical limit of bone loss that can lead to osteoporotic fractures.

Since calcium is an essential ingredient of bone, it is believed necessary to have adequate calcium intake either in the diet or in supplements. Without Vitamin D the calcium cannot be incorporated into the bone and therefore adequate Vitamin D intake is needed too.

### Milk and Milk Proteins

As well as being a good protein source, bovine milk is often the main dietary source of calcium and vitamin D. Milk proteins and their effects on bone disease have been the focus of a large amount of research. The proteins found in milk include immunoglobulins, growth factors, bovine serum albumin (BSA), alpha-lactalbumin, beta-lactoglobulin and a large number of caseins, all of which are phosphoproteins. These proteins, with the exception of casein, are also present in whey. Milk is known to contain a variety of mitogenic proteins and proteins which may be involved directly in bone remodelling.

Caseinoglycomacropeptide (CGMP) is a peptide released from kappa-casein during the rennet-mediated casein coagulation step (through the action of chymosin) of the cheese making process and is found in the whey fraction which is known as Sweet Whey or Cheese Whey. CGMP is sometimes referred to simply as GMP (glycomacropeptide). Cheese whey proteins consist of 15% to 20% CGMP. CGMP has been put forward as one of the bone health promoting components of milk, as disclosed in WO 00/49885 (discussed below).

Lactic acid whey is produced by fermentation with lactic acid bacteria or direct addition of lactic acid during the manufacture of caseinate or cottage and ricotta cheeses. Mineral acid whey is produced by addition of mineral acids during caseinate manufacture. Lactic acid whey and mineral acid whey do not contain CGMP. The basis of these two processes is to lower pH to about 4.6 to cause casein to precipitate as opposed to using the action of chymosin to cause precipitation.

Therefore any milk products that have not been exposed to chymosin will not contain CGMP.

Growth factors (IGF - Insulin-like Growth Factor, TGF - Transforming Growth Factor etc), immunoglobulins, BSA and some beta-lactoglobulin are recovered from milk or whey by cation exchange chromatography. Some growth factors are recovered as neutral proteins. CGMP is an acidic protein fraction recoverable by anion exchange.

Osteopontin (OPN) is a highly phosphorylated and glycosylated protein found in all body fluids (including milk) and in the extracellular matrix of mineralized tissues. OPN, one of the more abundant non-collagenous proteins in bone, is localized to cell-matrix and matrix-matrix interfaces in mineralized tissues, where it is deposited as the result of osteoclast action. OPN may protect the exposed bone surface or prime it for subsequent cell-matrix interactions. It has been proposed that OPN acts as an opsonin, facilitating macrophage adhesion and phagocytosis of particulate mineralized tissue debris. OPN can be cross linked by transglutaminase, and it can bind to various extracellular molecules including type I collagen, fibronectin and osteocalcin. This might be expected to add physical strength to extracellular matrices. OPN appears to promote the attachment of bone cells to bone matrix. OPN is present in vertebrate blood serum at relatively high concentrations and is thus also excreted in mammalian milk. (Denhardt, D.T. and Noda, M., Osteopontin expression and function: Role in bone remodelling, J. Cell Biochem. Suppl. 30/31:92-102 (1998)).

### Prior Art

The majority of published patents in the prior art have focused on the bone growth activity of basic proteins - ie those derived by the use of cation exchange.

New Zealand Patent Specification 503608 teaches of preparing a bone anti-resorption agent from milk or heated whey by cation exchange chromatography. Cystatin or enzyme hydrolysed products of cystatin are disclosed as being effective in suppressing bone resorption. Bone, bone and joint and periodontal disease may be prevented (or treated) by ingesting drinks, food products or feeds in which cystatin or its hydrolysed products are present.

New Zealand Patent Specification 282898 discloses bovine IGF-1 like growth factors that are purified by cation exchange chromatography from milk, skim milk, cheese whey, reconstituted whey, WPC, WPI, milk powder, whey powders or colostrum. These materials are preferably heated before the cation exchange step. The binding is done at a temperature between 4 and 40 degrees C using a defined protein to cation exchanger ratio. The growth promoting effects of the preparation were demonstrated in cultured osteoblast like cells (MC3T3-E1 cells). The composition is claimed to be useful for preventing or treating bone and articulation diseases, in particular osteoporosis. If administered to humans during their growth phase, their peak bone mass may be increased. As a raw ingredient, the claimed material is useful for incorporation in beverages, foods medicines and animal diets.

European Patent Specification EP 0787499 (corresponding to Japanese Patent Abstract 8045566) teaches that kininogen, found in bovine plasma and milk, promotes bone formation and inhibits bone resorption. Whole kininogen, the fragment 1.2 of kininogen and the enzymatically degraded products of kininogen (molecular weight range 0.1-70 kDa (kilo Daltons) are all claimed to be active. The patent covers uses in drinks, foods, medicines and feed for this family of products. A weakly basic cation exchanger is used to prepare kininogen.

New Zealand Patent Specification 314286 discloses N-terminal sequences of the High Mobility Group (HMG) protein and amphoterin, or their degradation products as bone-growth promoters and bone resorption inhibitors. The degradation products that are active have a molecular weight of 0.1-20 kDa. The claimed preparations can be used as components of food, drink, medicine or feed where calcium is included. HMG protein and amphotericin recovered from other body fluids also acts similarly. In the example given the HMG protein of milk is isolated by cation exchange chromatography and subsequently purified on an S-Sepharose and a Mono-Q column.

New Zealand Patent Specification 246211 teaches of an osteoblast growth/bone enhancing factor from whey that is obtained by precipitating acidified whey with ethanol and having a molecular weight of between 5-28 kDa. The active agent is recovered as a water extract of the ethanol-precipitated fraction. Alternately, the same factor can be recovered in the permeate when heated whey is ultrafiltered through a 30 kDa membrane. Isoelectric point of the preparation is between 4 and 9. No anion exchange chromatography is used.

New Zealand Patent Specification 314097 discloses a milk derived protein of molecular weight between 2 and 24 kDa with an isoelectric point of between 7.5-11.0 having an osteoblast proliferation effect, a bone strengthening effect and a bone resorption inhibiting effect. The active protein is recovered by cation exchange chromatography and is eluted with 0.1M-1.0M salt. Also claimed are food, drink, medicines and feeds containing such a preparation.

New Zealand Patent Specification 301362 (corresponding to Japanese Patent Abstract JP 207508) teaches of an osteoclast inhibiting protein, the DNA encoding it and a method for expression of the DNA. The protein is 60kDa under reducing conditions and from 60-120kDa under non-reducing conditions. The protein can be purified by cation exchange or by binding to a heparin column. The biological activity of the protein is decreased by heating at 56 degrees C for 10 minutes.

European Patent Specification EP 704218 discloses the preparation of a basic milk based protein fraction and a milk based basic peptide fraction. These are derived by cation exchange chromatography of milk or whey. Both products, and their hydrolysates, promote bone growth and suppress the resorption of osteoclasts when orally administered. Can be presented as a food or a drink product. The compositions described are claimed to be useful for treating or preventing various bone diseases such as osteoporosis.

PCT Patent Specification WO 00/49885 discloses a composition for prevention or treatment of a bone or dental disorder which comprises a milk protein hydrolysate. In preferred embodiments the milk protein hydrolysate is a hydrolysate of casein, in particular a caseinoglycomacropeptide (CGMP), a mimetic, homologue or fragment thereof in a bioavailable form which retains the ability of CGMP to inhibit bone resorption or bone loss; or favour calcium absorption, retention or calcification; or a combination thereof. The composition is produced from sweet whey by concentration and weak anion chromatography.

Bayless et al (Isolation and biological properties of osteopontin from bovine milk, Protein Expression and Purification 9(3): 309-314 (1997)) disclose a method for purifying osteopontin present in raw skim milk using DEAE-Sephacel (at the natural milk pH of 6.6) mixed overnight at 4 degrees C. The unbound fraction was removed and other bound proteins were removed with a 0.25M NaCl wash. An osteopontin containing fraction was recovered by a 0.3M elution. Osteopontin containing fractions were pooled, made 4M in salt and then purified by hydrophobic interaction chromatography on a phenyl Sepharose column (twice). Isolation from raw skim is not acceptable commercially and the focus is on a highly purified sample, not an enriched product stream.

An early study by Takada et al (Milk whey protein enhances the bone breaking force in ovariectomised rats, Nutrition Research 17, 1709-1720 (1997)) shows that bone strengthening components are present in whey. The active components are heat stable and are present in the low molecular weight, 30-70% ethanol-precipitable portion of whey. No fractionation of whey was done.

Yun S. S. et al (Isolation of mitogenic glycophosphopeptides from cheese whey-protein concentrate, Bioscience Biotechnology and Biochemistry, 60(3): 429-433 (1996)) investigated the immunological function of cheese whey protein concentrate (CWPC) using mitogenic activity in murine splenocytes as an index. A fraction isolated by gel filtration and anion exchange chromatography of CWPC showed high mitogenic activity. The study's results demonstrated that cheese whey contains a glycophosphopeptide (GPP) having strong mitogenic activity.

Sorensen E. S. et al (Purification and characterization of 3 proteins isolated from the proteose peptone fraction of bovine-milk, Journal of Dairy Research, 60(2):189-197 (1993)) isolated three major proteins from the proteose peptone of bovine milk. These were purified by Sephadex G-75 gel chromatography, Q-Sepharose ion-exchange and additional Sephadex G-75 gel chromatography in the presence of urea. From their mobility in a gradient SDS-PAGE the proteins were found to have molecular masses of 17, 28 and 60 kDa. The N-terminal amino acid sequence of the 17 kDa protein was found to be homologous with a camel whey protein. This protein had not previously been described in bovine milk. From the SDS-PAGE results, the 28 kDa protein was judged to be the major protein of proteose peptone, contributing approximately 25% of the total. The N-terminal amino acid sequence showed no homology to any known protein sequence, but the amino acid composition indicated that the 28 kDa protein is identical with the PP3 component from the proteose peptone fraction of bovine milk or part of it. The 60 kDa protein was found to be bovine osteopontin, a very highly phosphorylated protein with an Arg-Gly-Asp sequence which mediates cell attachment.

FitzGerald 998 (FitzGerald, R.G. "Potential uses of caseinophosphopeptides", In. Dairy Journal. (1998) 8:451-457) reports use of caseinophosphopeptides to enhance mineral bioavailability and increase absorption of calcium.

Sorenson and Peterson, 1993 (Sorenson, E.S. and Peterson, T.E., "Purification and characterization of three proteins isolated from the proteose peptone fraction of bovine milk", J. Diary Res. (1993) 60:189-197) reports purification of proteose peptone 3, osteopontin and a 17 kEa protein analogous with a camel whey protein from a crude proteose peptone fraction prepared by acidification and heating of skim milk followed by trichioro acetic acid and acetone precipitation and wash steps.

GB 2 188 526 A discloses the preparation of a proteinaceous material from whey produced by rennet coagulation of whole milk or acidification of milk.

Publications in the science and the patent literature have not shown that acidic fractions of milk (or whey) provide a (potential) source of a bone anti-resorption agent

### Objects of the Invention

It is therefore an object of different aspects of the invention to provide bone health compositions derived from an acidic protein fraction of milk and particularly from an acidic protein fraction of whey; methods of producing such compositions; methods of treatment comprising said compositions; medicinal uses of said compositions; and/or at the least to provide the public with a useful choice.

### SUMMARY OF THE INVENTION

According to the present invention, there is provided an ingestible composition comprising an acidic protein fraction of acid whey or a combination thereof wherein the composition does not contain caseinoglycomacropeptide (CGMP) and wherein the proteins in the fraction have a molecular weight distribution of 3,000 to 65,000 as measured by SDS-PAGE.

Preferably, the acid protein fraction comprises 70% by weight or more of proteins of which 80% by weight or more comprise osteopontin and proteose peptones.

Advantageously, the proteose peptones comprise peptides generated from casein by the action of plasmin and include one or more of the proteins selected from the group comprising proteose peptone 5 (PP5), proteose peptone 8-slow (PP8-slow), proteose peptone 8-fast (PP8-fast), as well as the non-casein proteose peptone 3 (PP3).

Advantageously, the sialic acid content of the fraction is in the range 0.8% to 6.5% and/or the phosphate content of the fraction is in the range 0.5% to 3%.

Conveniently, the composition is produced using anion exchange chromatography or strong anion exchange chromatography.

Preferably, the composition is derived from any one or more feedstocks selected from the group comprising recombined or fresh whole milk, recombined or fresh skim milk, reconstituted whole or skim milk powder, colostrum, milk protein concentrate (MPC), milk protein isolate (MPI), whey protein isolate (WPI), whey protein concentrate (WPC), whey, reconstituted whey powder, or derived from any milk processing stream, or derived from the permeates obtained by ultrafiltration and/or microfiltration of any one or more of these feedstocks.

Advantageously, the composition is derived from lactic acid whey or mineral acid whey.

The present invention also provides a method of producing an ingestible bone health composition comprising the steps of:
(a) providing an aqueous solution that does not contain CGMP and that is selected from the group comprising lactic acid whey, or mineral acid whey;
(b) subjecting the aqueous solution to anion exchange chromatography at a pH of from about pH 3 to about pH 4.9;
(c) washing the anion exchange medium;
(d) eluting from the anion exchange medium an acidic protein fraction of whey.

Advantageously, the anion exchange chromatography is strong anion exchange chromatography.

Conveniently, step (b) is carried out between pH 4 and pH 4.7.

Advantageously, step (b) is carried out at pH 4.5.

Advantageously, step (c) comprises use of de-mineralised water to wash the medium.

Conveniently, step (d) is carried out using a solution selected from the group comprising NaCl, KCI, a mixture of NaCl and KCl, an acid, two more eluting solutions having different pHs, and an eluting solution having a pH between 5.0 and 9.0 and a salt concentration up to 1.0 M.

Advantageously, the method further comprises a step or steps before step (a) wherein the step or steps comprise one or more steps selected from the group comprising thermisation, pasteurisation, centrifugation, ultrafiltration, microfiltration, reverse osmosis, electrodialysis, or ion exchange chromatography.

The present invention further provides a composition produced by the method of the invention.

Advantageously, the composition further comprises physiologically acceptable amounts of calcium, magnesium, vitamin C, vitamin D, vitamin E, vitamin K₂ and/or zinc.

Preferably, the composition is derived from one or a combination of bovine and other dairy sources, including goats or sheep or other milk producing mammals.

Advantageously, the composition comprises one or more compositions selected from the group comprising osteopontin, bone sialoprotein, proteose peptone 3, proteose peptone 5, proteose peptone 8, sialyated and phosphorylated proteins and peptides obtained therefrom, and alpha-s1-casein phosphopeptides.

Preferably, the composition is in the form of a dietary supplement, a food additive, a drink additive, a nutraceutical or a pharmaceutical.

The present invention further provides the use of an acidic protein fraction of milk or a combination thereof in the manufacture of an ingestible composition for maintaining or improving bone health, wherein the composition does not contain caseinoglycomacropeptide (CGMP).

Preferably, the use is for treating or preventing net bone loss.

Advantageously, the use is for treating, managing and/or preventing bone defects.

Preferably, the proteins in the fraction have the molecular weight distribution of 3,000 and 65,000 as measured by SDS-PAGE.

Advantageously, the acidic protein fraction comprises 70% by weight or more of proteins of which 80% by weight or more comprise osteopontin and preteose peptones.

Conveniently, the proteose peptones comprise peptides generated from casein by the action of plasmin and include one more of the proteins selected from the group comprising proteose peptone 5 (PP5), proteose peptone 8-slow (PP8-slow), proteose peptone 8-fast (PP8-fast), as well as the non-casein proteose peptone 3 (PP3).

Advantageously, the use of the invention utilises the composition according to the present invention.

The invention consists in the foregoing and also envisages constructions of which the following gives examples.

### BRIEF DESCRIPTION OF THE DRAWINGS

Figure 1 shows the HPLC Mono Q analysis of the acidic whey protein fraction derived from mineral acid whey in Example 1.
Figure 2 shows the HPLC Mono Q analysis of the acidic whey protein fraction derived from lactic acid whey in Example 2.
Figure 3 shows the anti-resorptive effect of the acidic whey protein fraction on mouse calvaria (the vault of the skull) cells in culture from Example 3.

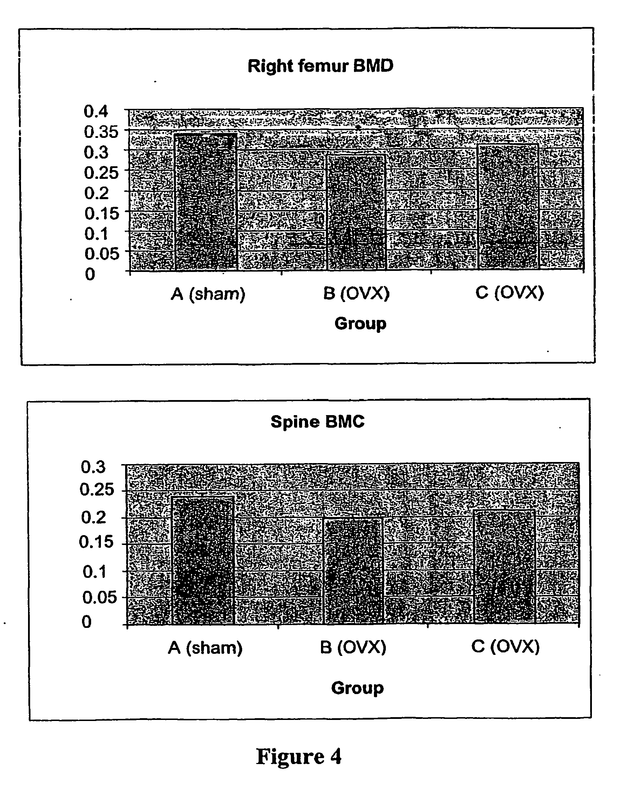

Figure 4 compares the bone mineral densities of the femurs and spines of the different groups of rats in the *in vivo* feeding trials from Example 4.

### DETAILED DESCRIPTION OF THE INVENTION

The Applicants have discovered that an acidic protein fraction of milk, of a component of milk, and particularly of whey, can reduce or prevent net bone loss.

The term "acidic protein fraction" is intended to mean a fraction of milk proteins comprising proteins that have an isoelectric point of 4.9 or less.

The acidic protein fraction of the invention has been shown to contain a number of minor acidic whey proteins. These include osteopontin, proteose peptone 3, proteose peptone 5 (PP5), also known as β-casein-5P(f1-105) or β-casein-5P(f1-107), proteose peptone 8-slow (PP8-slow), also known as β-casein-1P(f29-105) or β-casein-1P(f29-107), sialyated and phosphorylated proteins, alpha-s1-casein phosphopeptides, and also a mixture of peptides derived from these proteins by natural proteolysis. Very small amounts of lactosylated alpha-and beta-lactoglobulins, bovine serum albumin and immunoglobulins are also present.

The acidic protein fraction has been shown to contain peptides derived from these proteins generated by the hydrolytic action of the naturally occurring milk protease plasmin. In the specific case of an acidic protein fraction recovered from lactic acid whey, there are a wider range of peptides generated naturally by the action of lactic acid bacterial proteases, as well as by plasmin.

Using an animal model that mimics the effect of menopause on bone (ovariectomised rats - OVX rats), the acidic protein fraction of the invention was found to inhibit the bone resorption normally observed post-menopause that can eventually lead to diseases such as osteoporosis. The acidic protein fraction of the invention is thus useful as a means of treating or preventing diseases such as osteoporosis and osteo-arthritis.

The acidic protein fraction of the invention has an overall sialic acid content in the range of 0.8% to 6.5% and a phosphate content of between 0.5 and 3%.

The acidic protein fraction contains proteins and peptides with a wide range of molecular weights ranging from 3000 Daltons (hydrolysis products) to approximately 65,000 Daltons (proteose peptone 3 aggregates).

Potential feedstocks for commercial recovery of the acidic protein fraction of the invention include recombined or fresh whole milk, recombined or fresh skim milk, reconstituted whole or skim milk powder, colostrum, milk protein concentrate (MPC), milk protein isolate (MPI), whey protein isolate (WPI), whey protein concentrate (WPC), whey, reconstituted whey powder, or derived from any milk processing stream, or derived from the permeates obtained by ultrafiltration and/or microfiltration of any one or more of these feedstocks. Potential feedstocks exclude those derived from sweet whey. Any of these potential feedstocks may be derived from one or more of bovine and other dairy sources (for example goat or sheep or other milk producing mammals). Alternatively, potential feed stocks may be derived from any milk processing stream, such as those produced during the manufacture of Lactalbumin™ or TMP™ (Total Milk Protein) isolates. Methods suitable for the commercial production of whey are described by J G Zadow (Ed) "Whey and Lactose Processing" (Elsevier Applied Science, London and New York, 1992) and T Sienkiewicz and C Riedel (Eds) "Whey and whey utilisation" (Verlag, Germany, 1990).

An acidic protein fraction of the invention is produced using anion exchange chromatography and preferably strong anion exchange chromatography. Preferably the anion exchange chromatography is carried out at between about pH 3.0 and pH 4.9, more preferably between about pH 4.0 and pH 4.7 and even more preferably at pH 4.5. These pH conditions are optimal for recovery of the acidic protein fraction by anion exchange chromatography because in this range the active component or components are bound to the column and unwanted proteins are almost completely excluded in the unbound fraction. These include major whey proteins such as alpha-lactalbumin, beta-lactoglobulin and bovine serum albumin, which would serve only as diluents of the measured activity.

### Industrial Application

The compositions of the invention and the compositions produced by the methods of the invention may be used for the generation of functional foods by incorporation into food or drink, and for the treatment, management and/or prevention of bone defects (all age groups) including osteo-arthritis, osteoporosis and dental disorders.

It is envisaged that the compositions of the invention will be ingested on a daily basis as neutraceuticals or dietary supplements in order to delay or prevent the onset of debilitating bone disorders.

### EXAMPLES

### Example 1 Acidic Protein Fraction Derived from Mineral Acid Whey

A 20L solution of mineral acid whey protein concentrate (Alacen 342 - available from NZMP, Wellington, New Zealand) at 10% solids and pH 4.5 was passed through a 2L column of Q-Sepharose BB (Amrad Pharmacia, Australia) at a flow rate of 110ml/min. The column was washed with 5L of demineralised water and eluted with a 1.0M solution of sodium chloride (pH 6.0). Protein adsorption and elution was monitored by measuring the absorbance at 280nm.

The acidic protein fraction eluted from the column was concentrated approximately 6.25 fold using an Amicon 3K NMCO Spiral ultrafiltration unit (available from Millipore, USA). The concentrated protein retentate was dialysed against water and then freeze dried.

The dry product (56g recovered) had a content of 79% protein, less than 0.5% calcium, approximately 1.0% phosphorous and 6.0 % sialic acid. The amino acid composition of the eluted protein fraction is shown in Table 1.

**Table 1 - Amino acid profile of the acidic protein fraction from mineral acid whey**

| **Amino Acid** | **Content (%w/w)** |
|---|---|
| Aspartic acid | 8.19 |
| Serine | 6.22 |
| Glutamic acid | 17.7 |
| Glycine | 1.34 |
| Histidine | 2.22 |
| Arginine | 3.69 |
| Threonine | 4.83 |
| Alanine | 2.59 |
| Proline | 6.15 |
| Tyrosine | 1.88 |
| Valine | 4.18 |
| Lysine | 6.64 |
| Isoleucine | 5.70 |
| Leucine | 6.72 |
| Phenylalanine | 2.86 |

The acidic protein fraction was analysed for whey proteins and proteose peptones by reverse phase HPLC according to the method described by Elgar et al (Journal of Chromatography A, 878 (2000), pp183-196). An analytical anion exchange Mono Q column HR 5/5 (obtained from Amersham-Pharmacia Biotech, Australia) was used to determine the protein composition of the acidic protein fraction. This detected the presence of osteopontin, alpha-s1-casein phosphopeptides, proteose peptone 3, proteose peptone 5 and beta-lactoglobulin. Proteose peptone 8-slow co-elutes with PP5 so does not appear as a distinct peak. Peptides derived from these proteins by natural proteolysis were also detected, as shown by the broad peaks for each component and the presence of small peaks surrounding the major peaks.

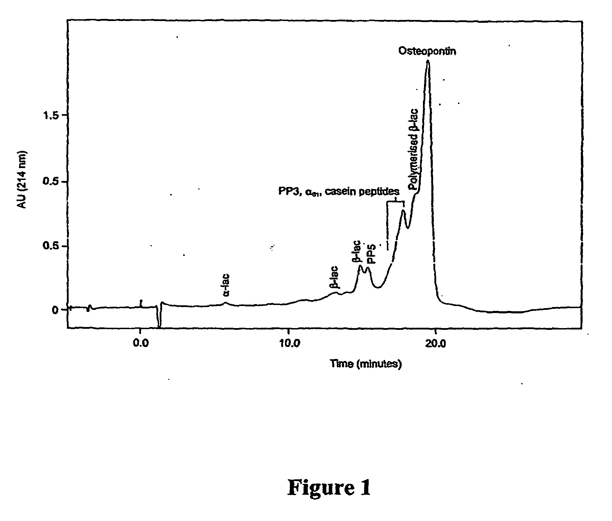

The freeze dried acidic fraction recovered from the anion exchanger was dissolved in 20mM Tris/HCl buffer, pH 8.0 and loaded onto the Mono Q column. The analytical separation was developed using a triphasic linear gradient to 1M sodium chloride (pH6.0). Protein adsorption and elution was measured at 214nm. Protein peak identities were resolved using standards prepared in our own laboratory. Standards were prepared for PP5, PP3 and osteopontin. The identity of each standard was confirmed by amino acid sequencing (such as N-terminal sequencing) and their purity by SDS-PAGE and HPLC analysis. Other components were identified by amino acid sequence analysis of peaks trapped from the HPLC run shown in Figure 1.

The results of these analyses (Figure 1) showed that osteopontin, alpha-sl-casein fragments, sialyated and/or phosphorylated minor proteins, proteose peptones 5 and 3, and peptides derived from these proteins were present in the acidic protein fraction recovered from mineral acid whey.

### Example 2 Acidic Protein Fraction Derived from Lactic Acid Whey

A 20L solution of lactic acid whey protein concentrate (Alacen 312 - available from NZMP, Wellington, New Zealand) at 10% solids and pH 4.5 was passed through a 2L column of Q-Sepharose BB at a flow rate of 110ml/min. The column was washed with 5L of demineralised water and eluted with a 1.0M solution of sodium chloride (pH 6.0). Protein adsorption and elution was monitored by measuring the absorbance at 280nm.

The protein eluted from the column was concentrated approximately 6.25 fold using an Amicon 3K NMCO Spiral ultrafiltration unit (available from Millipore, USA). The concentrated protein retentate was dialysed against water and then freeze dried.

The acidic protein fraction was analysed for whey proteins and proteose peptones by reverse phase HPLC according to the method described by Elgar et al (Journal of Chromatography A, 878 (2000), pp183-196). An analytical anion exchange Mono Q column HR 5/5 (obtained from Amersham-Pharmacia Biotech, Australia) was used to determine the presence of osteopontin, alpha-s1-casein phosphopeptides, proteose peptone 3 and proteose peptone 5. The presence of peptides derived from these proteins was also observed as discussed in Example 1.

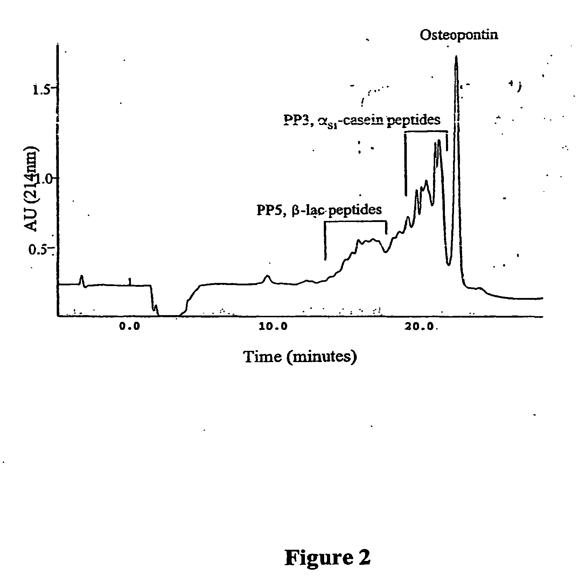

The freeze dried acidic protein fraction recovered from the anion exchanger was dissolved in 20mM Tris/HCl buffer, pH 8.0 and loaded onto the Mono Q column. The analytical separation was developed using a triphasic linear gradient to 1M sodium chloride (pH 6.0). Protein adsorption and elution was measured at 214nm. Protein peak identities were resolved using known standards or N-terminal sequencing of trapped peaks. The results of these analyses (Figure 2) showed that osteopontin, alpha-s1-casein fragments, sialyated and/or phosphorylated minor proteins, proteose peptones 5 and 3 and peptides derived from these proteins, were present in the acid fraction recovered from lactic acid whey. Figure 2, in comparison with.Figure 1, shows the presence of a larger number of peptides derived from the proteins of the acidic protein fraction.

### Example 3 In Vitro Analysis of Efficacy

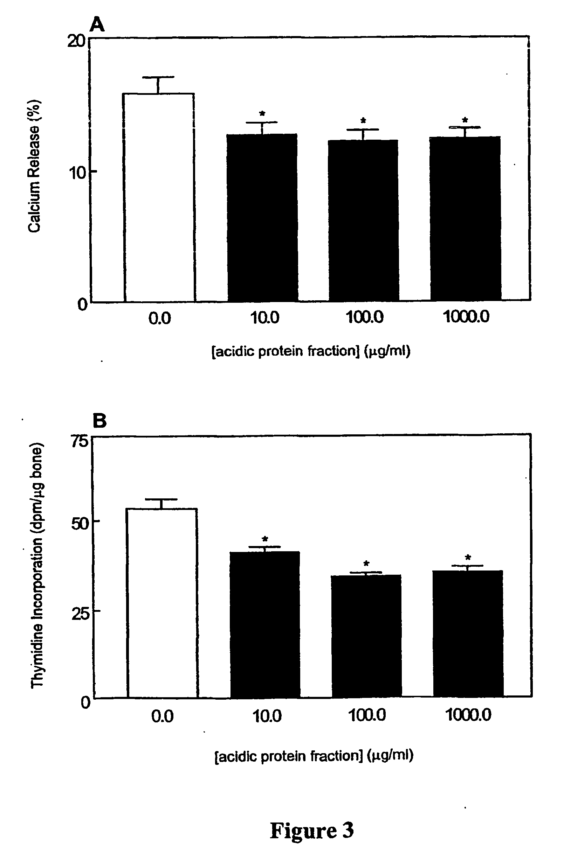

The product from Example 1 was tested in the bone organ culture model as described by Lowe et al (Journal of Bone and Mineral Research (US), 6(12):1277-1283 (1991)). Figure 3 shows that the acidic protein fraction had anti-resorptive effects on the cells from the bone organ culture at doses as low as 10ug/ml. Both lower calcium release and lower thymidine incorporation compared to the controls demonstrate the anti-resorptive effect.

### Example 4 In Vivo Analysis of Efficacy

The ability of the acidic protein fraction from Example 1 to reduce bone loss induced by oestrogen deficiency in the ovariectomised (OVX) rat was studied over a 16 week period. The ovariectomised rat model is a widely accepted model for studying the bone loss that occurs post-menopause.

Thirty 6-month-old female Sprague-Dawley rats were received as 10 Sham-operated animals and 20 OVX animals at age 5.5 months. Sham operated animals undergo anaesthesia and an incision is made but the ovaries are left intact. In the OVX animals the ovaries are removed. On arrival animals were separated into three groups (n=10 per group). These groups are shown in Table 2.

## Claims

1. An ingestible composition comprising an acidic protein fraction of acid whey, hydrolysates of an acidic protein fraction of acid whey or a combination thereof wherein the composition does not contain caseinoglycomacropeptide (CGMP) and wherein the proteins in the fraction have a molecular weight distribution of 3,000 to 65,000 as measured by SDS-PAGE.

2. A composition of claim 1 wherein the acidic protein fraction comprises 70% by weight or more of proteins of which 80% by weight or more comprise osteopontin and proteose peptones.

3. A composition of claim 2 wherein the proteose peptones comprise peptides generated from casein by the action of plasmin and include one or more of the proteins selected from the group comprising proteose peptone 5 (PP5), proteose peptone 8-slow (PP8-slow), proteose peptone 8-fast (PP8-fast), as well as the non-casein proteose peptone 3 (PP3).

4. A composition of any one of claims 1 to 3 wherein the sialic acid content of the fraction is in the range 0.8% to 6.5% and/or the phosphate content of the fraction is in the range 0.5% to 3%.

5. A composition of any one of claims 1 to 4 wherein the composition is produced using anion exchange chromatography or strong anion exchange chromatography.

6. A composition of any one of claims 1 to 5 derived from any one or more feedstocks selected from the group comprising recombined or fresh whole milk, recombined or fresh skim milk, reconstituted whole or skim milk powder, colostrums, milk protein concentrate (MPC), milk protein isolate (MPI), whey protein isolate (WPI), whey protein concentrate (WPC), whey, reconstituted whey powder, or derived from any milk processing stream, or derived from the permeates obtained by ultrafiltration and/or microfiltration of any one or more of these feedstocks.

7. A composition of any one of claims 1 to 6 derived from lactic acid whey or mineral acid whey.

8. A method of producing an ingestible bone health composition comprising the steps of:
(a) providing an aqueous solution that does not contain CGMP and that is selected from the group comprising lactic acid whey or mineral acid whey;
(b) subjecting the aqueous solution to anion exchange chromatography at a pH of from about pH 3 to pH 4.9;
(c) washing the anion exchange medium;
(d) eluting from the anion exchange medium an acidic protein fraction of whey.

9. A method of claim 8 wherein the anion exchange chromatography is strong anion exchange chromatography.

10. A method of any one of claims 8 to 9 wherein step (b) is carried out between pH 4 and pH 4.7.

11. A method of claim 10 wherein step (b) is carried out at pH 4.5.

12. A method of any one of claims 8 to 11 wherein step (c) comprises use of de-mineralised water to wash the medium.

13. A method of any of claims 8 to 12 wherein step (d) is carried out using a solution selected from the group comprising NaCl, KCl, a mixture of NaCl and KCl, an acid, two more eluting solutions having different pHs, and an eluting solution having a pH between 5.0 and 9.0 and a salt concentration up to 1.0 M.

14. A method of any one of claims 8 to 13 further comprising a step or steps before step (a) wherein the step or steps comprise one or more steps selected from the group comprising thermisation, pasteurisation, centrifugation, ultrafiltration, microfiltration, reverse osmosis, electrodialysis, or ion exchange chromatography.

15. A composition produced by the method of any one of claims 8 to 14.

16. A composition of any one of claims 1 to 7 and 15 further comprising physiologically acceptable amounts of calcium, magnesium, vitamin C, vitamin D, vitamin E, vitamin K₂ and/or zinc.

17. A composition of any one of claims 1 to 7, 15 and 16 wherein the composition is derived from one or a combination of bovine and other dairy sources, including goats or sheep or other milk producing mammals.

18. A composition of any one of claims 1 to 7 and 15 to 17 comprising one or more compositions selected from the group comprising osteopontin, bone sialoprotein, proteose peptone 3, proteose peptone 5, proteose peptone 8, sialyated and phosphorylated proteins and peptides obtained therefrom, and alpha-s1-casin phosphopeptides.

19. A composition of any one of claims 1 to 7 and 15 to 18 in the form of a dietary supplement, a food additive, a drink additive, a nutraceutical or a pharmaceutical.

20. A use of an acidic protein fraction of milk, hydrolysates of an acidic protein fraction of milk or a combination thereof in the manufacture of an ingestible composition for maintaining or improving bone health, wherein the composition does not contain caseinoglycomacropeptide (CGMP).

21. A use of claim 20 for treating or preventing net bone loss.

22. A use of claim 20 for treating, managing and/or preventing bone defects.

23. A use of any one of claims 20 to 22 wherein the proteins in the fraction have a molecular weight distribution of 3,000 to 65,000 as measured by SDS-PAGE.

24. A use of any one of claims 20 to 23 wherein the acidic protein fraction comprises 70% by weight or more of proteins of which 80% by weight or more comprise osteopontin and proteose peptones.

25. A use of claim 24 wherein the proteose peptones comprise peptides generated from casein by the action of plasmin and include one or more of the proteins selected from the group comprising proteose peptone 5 (PP5), proteose peptone 8-slow (PP8-slow), proteose peptone 8-fast (FF8-fast), as well as the non-casein proteose peptone 3 (PP3).

26. A use of claim 20, 21 or 22 wherein the composition comprises the composition of any one of claims 1 to 7 and 15 to 19.

## Patentansprüche

1. Einnehmbare Zusammensetzung, eine saure Proteinfraktion von Sauermolke, Hydrolysate einer sauren Proteinfraktion von Sauermolke oder eine Kombination daraus umfassend, wobei die Zusammensetzung kein Caseinglycomakropeptid (CGMP) enthält und wobei die Proteine der Fraktion eine mit SDS-PAGE gemessene Molmassenverteilung von 3.000 bis 65.000 aufweisen.

2. Zusammensetzung nach Anspruch 1, wobei die saure Proteinfraktion 70 Gew.-% oder mehr Proteine umfasst, von denen 80 Gew.-% oder mehr Osteopontin und Proteose-Peptone umfassen.

3. Zusammensetzung nach Anspruch 2, wobei die Proteose-Peptone Peptide umfassen, die durch die Einwirkung von Plasmin aus Casein erzeugt sind, und eines oder mehrere der Proteine umfassen, die aus der Gruppe ausgewählt sind, die Proteose-Pepton 5 (PP5), Proteose-Pepton 8-langsam (PP8-langsam), Proteose-Pepton-8-schnell (PP8-schnell) sowie das Nichtcasein-Preoteose-Pepton 3 (PP3) umfasst.

4. Zusammensetzung nach einem der Ansprüche 1 bis 3, wobei der Sialinsäuregehalt der Fraktion im Bereich von 0,8 % bis 6,5 % und/oder der Phosphatgehalt der Fraktion im Bereich von 0,5 % bis 3 % liegen.

5. Zusammensetzung nach einem der Ansprüche 1 bis 4, wobei die Zusammensetzung mit Hilfe von Anionenaustauschchromotographie oder starker Anionenaustauschchromatographie hergestellt wird.

6. Zusammensetzung nach einem der Ansprüche 1 bis 5, die von einem beliebigen Rohmaterial oder mehreren Rohmaterialien stammt, die aus der Gruppe ausgewählt sind, die rekombinierte oder frische Vollmilch, rekombinierte oder frische Magermilch, rekonstituiertes Voll- oder Magermilchpulver, Kolostrum, Milchproteinkonzentrat (MPC), Milchproteinisolat (MPI), Molkeproteinisolat (WPI), Molkeproteinkonzentrat (WPC), Molke, rekonstituiertes Molkepulver umfasst, oder aus einem beliebigen Milchverarbeitungsablauf stammt oder aus den Permeaten stammen, die durch Ultrafiltration und/oder Mikrofiltration eines oder mehrerer dieser Rohstoffe gewonnen werden.

7. Zusammensetzung nach einem der Ansprüche 1 bis 6, die aus Milchsäure-Molke oder Mineralsäure-Molke stammt.

8. Verfahren zum Herstellen einer einnehmbaren Knochengesundheits-Zusammensetzung, folgende Schritte umfassend:
(a) Bereitstellen einer wässrigen Lösung, die kein CGMP enthält und die aus der Gruppe ausgewählt ist, die Milchsäure-Molke oder Mineralsäure-Molke umfasst,
(b) Unterwerfen der wässrigen Lösung einer Anionenaustauschchromatographie bei einem pH-Wert von etwa 3 bis 4,9,
(c) Waschen des Anionenaustauschmediums,
(d) Eluieren einer sauren Proteinfraktion von Molke aus dem Anionenaustauschmedium.

9. Verfahren nach Anspruch 8 wobei die nionenaustauschchromatographie die starke Anionenaustauschchromatographie ist.

10. Verfahren nach einem der Ansprüche 8 bis 9, wobei Schritt (b) bei einem pH-Wert zwischen 4 und 4,7 durchgeführt wird.

11. Verfahren nach Anspruch 10, wobei Schritt (b) bei einem pH-Wert von 4,5 durchgeführt wird.

12. Verfahren nach einem der Ansprüche 8 bis 11, wobei Schritt (c) die Verwendung entmineralisierten Wassers zum Waschen des Mediums umfasst.

13. Verfahren nach einem der Ansprüche 8 bis 12, wobei Schritt (d) unter Verwendung einer Lösung durchgeführt wird, die aus der Gruppe ausgewählt ist, die NaCl, KCI, ein Gemisch von NaCl und KCI, eine Säure, zwei weitere Elutionslösungen mit unterschiedlichen pH-Werten sowie eine Elutionslösung mit einem pH-Wert zwischen 5,0 und 9,0 und einer Salzkonzentration bis zu 1,0 M umfasst.

14. Verfahren nach einem der Ansprüche 8 bis 13, ferner vor Schritt (a) einen Schritt oder Schritte umfassend, wobei der Schritt oder die Schritte einen oder mehrere Schritte umfassen, der/die aus der Gruppe ausgewählt ist/sind, die Thermisierung, Pasteurisierung, Zentrifugation, Ultrafiltration, Mikrofiltration, Umkehrosmose, Elektrodialyse und Ionenaustauschchromatographie umfasst.

15. Zusammensetzung, die mit dem Verfahren nach einem der Ansprüche 8 bis 14 hergestellt ist.

16. Zusammensetzung nach einem der Ansprüche 1 bis 7 und 15, ferner physiologisch unbedenkliche Mengen von Calcium, Magnesium, Vitamin C, Vitamin D, Vitamin E, Vitamin K2 und/oder Zink umfassend.

17. Zusammensetzung nach einem der Ansprüche 1 bis 7, 15 und 16, wobei die Zusammensetzung aus einer Kuh- oder anderen Milchquelle stammt, einschließlich Ziege, Schaf und anderer milchproduzierender Säuger, oder einer Kombination aus diesen.

18. Zusammensetzung nach einem der Ansprüche 1 bis 7 und 15 bis 17, eine oder mehrere Zusammensetzungen umfassend, die aus der Gruppe ausgewählt sind, die Osteopontin, Knochensialoprotein, Proteose-Pepton 3, Proteose-Pepton 5, Proteose-Pepton 8, daraus erhaltene sialyierte und phosphorylierte Proteine und Peptide und alpha-s1-Caseinphosphopeptide umfasst.

19. Zusammensetzung nach einem der Ansprüche 1 bis 7 und 15 bis 18 in der Form einer Nahrungsergänzung, eines Nahrungsmittelzusatzes, eines Getränkezusatzes, eines Nutrazeutikums oder eines Pharmazeutikums.

20. Verwendung einer sauren Proteinfraktion von Milch, Hydrolysaten einer sauren Proteinfraktion von Milch oder einer Kombination davon bei der Herstellung einer einnehmbaren Zusammensetzung zum Aufrechterhalten oder Verbessern der Knochengesundheit, wobei die Zusammensetzung kein Caseinglycomakropeptid (CGMP) enthält.

21. Verwendung nach Anspruch 20 zum Behandeln oder Vorbeugen von Knochenverlust.

22. Verwendung nach Anspruch 20 zum Behandeln, Bewältigen und/oder Vorbeugen von Knochendefekten.

23. Verwendung nach einem der Ansprüche 20 bis 22, wobei die Proteine in der Fraktion eine mit SDS-PAGE gemessene Molmassenverteilung von 3.000 bis 65.000 aufweisen.

24. Verwendung nach einem der Ansprüche 20 bis 23, wobei die saure Proteinfraktion 70 Gew.-% oder mehr Proteine umfasst, von denen 80 Gew.-% oder mehr Osteopontin und Proteose-Peptone umfassen.

25. Verwendung nach Anspruch 24, wobei die Proteose-Peptone Peptide umfassen, die durch die Einwirkung von Plasmin aus Casein erzeugt sind und eines oder mehrere der Proteine umfassen, die aus der Gruppe ausgewählt sind, die Proteose-Pepton 5 (PP5), Proteose-Pepton-8-langsam (PP8-langsam), Proteose-Pepton-8-schnell (PP8-schnell) sowie das Nichtcasein-Proteose-Pepton 3 (PP3) umfasst.

26. Verwendung nach Anspruch 20, 21 oder 22, wobei die Zusammensetzung die Zusammensetzung nach einem der Ansprüche 1 bis 7 und 15 bis 19 umfasst.

## Revendications

1. Une composition ingérable comprenant une fraction de protéine acide de lactosérum acide, des hydrolysates d'une fraction de protéine acide de lactosérum acide ou une combinaison de ces éléments, dans laquelle la composition ne contient pas de caséinoglycomacropeptide (CGMP) et dans laquelle les protéines de la fraction possèdent une répartition des poids moléculaires mesurée par SDS-PAGE de 3 000 à 65 000.

2. Une composition selon la revendication 1, dans laquelle la fraction de protéine acide comprend 70 % par poids ou plus de protéines, dont 80 % par poids ou plus comprennent de l'ostéopontine et des protéoses-peptones.

3. Une composition selon la revendication 2, dans laquelle les protéoses-peptones comprennent des peptides générés à partir de la caséine par l'action de la plasmine et comprennent une ou plusieurs des protéines sélectionnées dans le groupe se composant de la protéose-peptone 5 (PP5), de la protéose-peptone 8-lente (PP8-lente), de la protéose-peptone 8-rapide (PP8-rapide), ainsi que la protéose-peptone non caséinique 3 (PP3).

4. Une composition selon n'importe laquelle des revendications 1 à 3, dans laquelle la teneur en acide sialique de la fraction va de 0,8 % à 6,5 % et/ou la teneur en phosphate de la fraction va de 0,5 % à 3 %.

5. Une composition selon n'importe laquelle des revendications 1 à 4, dans laquelle la composition est produite par chromatographie d'échange anionique ou par chromatographie d'échange anionique forte.

6. Une composition selon n'importe laquelle des revendications 1 à 5 dérivée d'une ou plusieurs matières premières sélectionnées parmi les suivantes : lait entier frais ou recombiné, lait écrémé frais ou recombiné, lait entier ou écrémé en poudre reconstitué, colostrums, concentré de protéines de lait, isolat de protéines de lait, isolat de protéines de lactosérum, concentré de protéines de lactosérum, lactosérum, lactosérum en poudre reconstitué, ou dérivée de toute opération de transformation du lait ou dérivée des perméats obtenus par ultrafiltration et/ou microfiltration d'une ou plusieurs de ces matières premières.

7. Une composition selon n'importe laquelle des revendications 1 à 6 dérivée du lactosérum d'acide lactique ou du lactosérum d'acide minéral.

8. Un procédé de production d'une composition ingérable pour la santé des os se composant des opérations suivantes :
(a) se procurer une solution aqueuse qui ne contient pas de CGMP et qui est sélectionnée parmi le lactosérum acide lactique et le lactosérum acide minéral ;
(b) soumettre la solution aqueuse à la chromatographie d'échange anionique à un pH allant d'environ 3 % à 4,9 % ;
(c) laver le support d'échange anionique ;
(d) éluer du support d'échange anionique une fraction de protéine acide de lactosérum.

9. Un procédé selon la revendication 8, dans lequel la chromatographie d'échange anionique est une chromatographie d'échange anionique forte.

10. Un procédé selon n'importe laquelle des revendications 8 à 9, dans lequel l'opération (b) a lieu entre un pH de 4 et un pH de 4,7.

11. Un procédé selon la revendication 10, dans lequel l'opération (b) a lieu à un pH de 4,5.

12. Un procédé selon n'importe laquelle des revendications 8 à 11, dans lequel l'opération (c) suppose d'utiliser de l'eau déminéralisée pour laver le support.

13. Un procédé selon n'importe laquelle des revendications 8 à 12, dans lequel l'opération (d) a lieu à l'aide d'une solution sélectionnée dans le groupe se composant de NaCl, KCI, un mélange de NaCl et de KCI, un acide, deux autres solutions d'élution ayant des pH différents et une solution d'élution ayant un pH situé entre 5,0 et 9,0 et une concentration de sel allant jusqu'à 1,0 M.

14. Un procédé selon n'importe laquelle des revendications 8 à 13, supposant également une ou plusieurs opérations avant l'opération (a), dans lequel la ou les opérations comprennent une ou plusieurs opérations sélectionnées parmi les suivantes : thermisation, pasteurisation, centrifugation, ultrafiltration, microfiltration, osmose inverse, électrodialyse ou chromatographie d'échange ionique.

15. Une composition obtenue selon le procédé décrit à n'importe laquelle des revendications 8 à 14.

16. Une composition selon n'importe laquelle des revendications 1 à 7 et 15, comprenant également des quantités physiologiquement acceptables de calcium, de magnésium, de vitamine C, de vitamine D, de vitamine E, de vitamine K₂ et/ou de zinc.

17. Une composition selon n'importe laquelle des revendications 1 à 7, 15 et 16, dans laquelle la composition est dérivée d'une ou d'une combinaison de sources bovines et autres sources laitières, y compris les chèvres ou les brebis ou d'autres mammifères producteurs de lait.

18. Une composition selon n'importe laquelle des revendications 1 à 7 et 15 à 17, comprenant une ou plusieurs compositions sélectionnées parmi ce qui suit :
ostéopontine, sialoprotéine osseuse, protéose-peptone 3, protéose-peptone 5, protéose-peptone 8, protéines sialylées et phosphorylées et peptides en provenant et phosphopeptides alpha-s1-caséine.

19. Une composition selon n'importe laquelle des revendications 1 à 7 et 15 à 18, se présentant sous la forme de complément alimentaire, d'additif pour aliments, d'additif pour boissons, de nutraceutique ou de pharmaceutique.

20. Une utilisation d'une fraction de protéine acide du lait, des hydrolysates d'une fraction de protéine acide du lait ou d'une combinaison de ces éléments dans la fabrication d'une composition ingérable pour maintenir ou améliorer la santé osseuse, dans laquelle la composition ne contient pas de caséinoglycomacropeptide (CGMP).

21. Une utilisation selon la revendication 20, destinée au traitement ou à la prévention de la perte osseuse nette.

22. Une utilisation selon la revendication 20, destinée au traitement, à la gestion et/ou à la prévention des défauts osseux.

23. Une utilisation selon les revendications 20 à 22, dans laquelle les protéines de la fraction possèdent une répartition des poids moléculaires mesurée par SDS-PAGE de 3 000 à 65 000.

24. Une utilisation selon les revendications 20 à 23, dans laquelle la fraction de protéine acide comprend 70 % par poids ou plus de protéines, dont 80 % par poids ou plus comprennent de l'ostéopontine et des protéoses-peptones.

25. Une utilisation selon la revendication 24, dans laquelle les protéoses-peptones comprennent des peptides générés à partir de la caséine par l'action de la plasmine et comprennent une ou plusieurs des protéines sélectionnées dans le groupe se composant de la protéose-peptone 5 (PP5), de la protéose-peptone 8-lente (PP8-lente), de la protéose-peptone 8-rapide (PP8-rapide), ainsi que la protéose-peptone non caséinique 3 (PP3).

26. Une utilisation selon les revendications 20, 21 ou 22, dans laquelle la composition comprend la composition de n'importe laquelle des revendications 1 à 7 et 15 à 19.
